# EUROPÄISCHE PATENTSCHRIFT

(11) **EP 0 693 954 B1**
(45) Veröffentlichungstag und Bekanntmachung des Hinweises auf die Patenterteilung: **07.07.1999**
(21) Anmeldenummer: 94911067.0
(22) Anmeldetag: 22.03.1994
(51) Int. Cl.: A61N 5/00, A61N 7/02, A61B 17/22

(54) **THERAPIEEINRICHTUNG ZUR BEHANDLUNG VON LEIDEN DES HERZENS UND HERZNAHER GEFÄSSE**
THERAPEUTIC APPLIANCE FOR THE TREATMENT OF CONDITIONS OF THE HEART AND OF BLOOD VESSELS IN THE VICINITY OF THE HEART
DISPOSITIF POUR LE TRAITEMENT DE MALADIES DU COEUR ET DE VAISSEAUX PROCHES DU COEUR

(30) Priorität: 15.04.1993 DE 4312264
(43) Veröffentlichungstag der Anmeldung: 31.01.1996
(73) Patentinhaber: SIEMENS AKTIENGESELLSCHAFT, 80333 München (DE)
(72) Erfinder: REICHENBERGER, Helmut, D-90542 Eckental (DE)
(86) Internationale Anmeldenummer: DE9400327
(87) Internationale Veröffentlichungsnummer: WO9423793

(56) Entgegenhaltungen:
- DE-A- 3 038 445
- DE-A- 3 150 513
- DE-A- 3 900 433
- DE-A- 3 932 967
- DE-A- 4 207 463

## Beschreibung

Die Erfindung betrifft eine Therapieeinrichtung zur Behandlung von Leiden des Herzens und Gefäßen, einschließlich herznaher Gefäße, mit einen Wirkbereich aufweisenden therapeutischen Ultraschallwellen, welche Therapieeinrichtung eine therapeutische Ultraschallquelle aufweist, die therapeutische Ultraschallwellen einer solchen Intensität erzeugt, daß in dem im Wirkbereich befindlichen Gewebe Gewebeveränderungen durch thermische Wirkung hervorgerufen werden, und die mehrere therapeutische Ultraschallwandler enthält, welche derart ausrichtbar sind, daß sich die von ihnen ausgehenden Ultraschallwellen in dem jeweils zu behandelnden Bereich überlagern.

Eine Einrichtung der eingangs genannten Art ist aus der DE-A-31 50 513 bekannt. Die Quelle dieser Therapieeinrichtung ist nur zur extrakorporalen Applikation geeignet.

Außerdem ist aus der DE 30 38 445 A1 eine Einrichtung bekannt, die eine in einen in den Ösophagus (Speiseröhre) einführbaren Katheter integrierte Stoßwellenquelle enthält. Die von dieser erzeugten fokussierten Stoßwellen werden in das Herz eingeleitet, um dieses zu stimulieren.

Eine aus der DE 39 00 433 A1 bekannte Einrichtung enthält eine oder mehrere extrakorporal applizierbare Ultraschallquellen. Die durch diese erzeugten therapeutischen Ultraschallwellen dienen der Behandlung von Sklerosen, z.B. im Bereich der Herzkranzgefäße, und zwar soll durch die therapeutischen Ultraschallwellen eine Rückemulgierung der in den sklerotischen Bereichen an der Gefäßwand abgelagerten Lipide bewirkt werden.

Mittels der bekannten Therapieeinrichtungen ist es zwar möglich, beispielsweise im Falle von Herzrhythmusstörungen oder Sklerosen auf die jeweiligen Symptome Einfluß zu nehmen. Es besteht jedoch in der Regel keine Möglichkeit, fokale pathogene Gebiete, wie beispielsweise akzessorische Bündel im Falle des WPW-Symptoms, oder Triggerzonen für Arrhythmien, bleibend zu beeinflussen.

Der Erfindung liegt die Aufgabe zugrunde, eine Therapieeinrichtung der eingangs genannten Art so auszubilden, daß die dauerhafte Beeinflussung pathogener Gebiete, insbesondere pathogener Herde, möglich ist.

Nach der Erfindung wird diese Aufgabe dadurch gelöst, daß die Ultraschallquelle derart dimensioniert ist, daß sie transösophageal applizierbar ist. Es besteht somit die Möglichkeit, fokale pathogene Gebiete durch Beaufschlagung mit therapeutischen Ultraschallwellen auszuschalten. In diesem Zusammenhang ist es besonders vorteilhaft, wenn die Intensität der therapeutischen Ultraschallwellen ausreicht, um Nekrotisierungen des im Wirkbereich befindlichen Herzgewebes hervorzurufen, da dann eine sichere Ausschaltung der fokalen pathogenen Gebiete gewährleistet ist. Da die Ultraschallquelle derart dimensioniert ist, daß sie transösophageal (durch die Speiseröhre bekannt) applizierbar ist, ist das innerhalb der Rippen befindliche Herz anders als im Falle extrakorporal erzeugter therapeutischer Ultraschallwellen ohne weiteres für die therapeutischen Ultraschallwellen zugänglich. Die Ultraschallquelle weist im Interesse einer kurzen Behandlungsdauer mehrere therapeutische Ultraschallwandler auf, die derart ausrichtbar sind, daß sich die von ihnen ausgehenden Ultraschallwellen in dem jeweils zu behandelnden Bereich überlagern, weil die Abmessungen, die ein Wandlerelement einer transösophageal applizierbaren Ultraschallquelle aus anatomischen Gründen maximal aufweisen kann, es im Falle der Verwendung eines einzigen Ultraschallwandlers nämlich nicht unter allen Umständen erlauben, dem jeweils zu behandelnden Bereich in hinreichend kurzer Zeit die zur Erzielung des Behandlungserfolges erforderliche Ultraschallenergie zuzuführen.

Es sei darauf hingewiesen, daß aus der EP 0 404 121 A1 und der US 5 036 855 Therapieeinrichtungen bekannt sind, die es gestatten, Tumorgewebe durch thermische Wirkung von Ultraschall zu behandeln. Dagegen sollen jedoch im Falle der aus der DE 39 00 433 A1 bekannten Einrichtung bei der Behandlung von Gefäßen und des Herzens bleibende Gewebeveränderungen durch die thermische Wirkung des Ultraschalls durch gepulste Abstrahlung des Ultraschalls gerade vermieden werden.

Infolge der anatomisch günstigen Lage des Ösophagus und des Herzens relativ zueinander, ist es zweckmäßig, wenn die Ultraschallquelle einen diagnostischen Ultraschallwandler enthält, der Bestandteil einer Ultraschall-Ortungseinrichtung zur Ortung des jeweils zu behandelnden Bereiches ist, da dann eine besonders gute Bildqualität der mittels der Ultraschall-Ortungseinrichtung erzeugten Ultraschallbilder gewährleistet ist.

Um die therapeutische Ultraschallquelle leicht in den Ösophagus einführen zu können, ist gemäß einer Variante der Erfindung vorgesehen, daß die therapeutischen Ultraschallwandler und gegebenenfalls der diagnostische Ultraschallwandler Bestandteile einer arretierbaren, kettenartigen Struktur sind, wobei die Struktur in nicht arretiertem Zustand flexibel und in arretiertem Zustand wenigstens im wesentlichen starr ist. Es wird also deutlich, daß infolge der im nicht arretierten Zustand gegebenen Flexibilität die Ultraschallquelle leicht appliziert werden kann, während zur Behandlung infolge der im arretierten Zustand vorliegenden Starrheit eine definierte Zuordnung der Elemente der Ultraschallquelle und des Herzens bzw. der zu behandelnden Gefäße relativ zueinander gegeben ist. Ein kettenartig aufgebauter diagnostischer Ultraschallapplikator, der zur Sonographie des Herzens transösophageal applizierbar ist, ist übrigens in "Transösophageale Echo Computer Tomographie", Wollschläger et al, Biomedizinische Technik, Band 34, Ergänzungsband, Seite 10, 1989, beschrieben.

Da sich die einzelnen Bereiche des Herzens bzw. die Blutgefäße infolge der Herztätigkeit relativ zu der in dem Ösophagus aufgenommenen Ultraschallquelle verlagern, sind gemäß einer bevorzugten Variante der Erfindung Mittel zur Detektion der Herztätigkeit vorgesehen, die eine Abgabe therapeutischer Ultraschallwellen nur während solcher Phasen der Herztätigkeit freigeben, in denen sich das Herz und die Gefäße in relativer mechanischer Ruhe befindet. Es ist dann sichergestellt, daß nur diejenigen Bereiche des Herzens bzw. der Gefäße einer Behandlung unterzogen werden, die dieser wirklich bedürfen.

Eine besonders vorteilhafte Ausführungsform der Erfindung sieht vor, daß zumindest eine extrakorporal, insbesondere inter- oder subkostal, d.h. zwischen den Rippen oder unter dem Rippenbogen, applizierbare therapeutische Ultraschallquelle vorgesehen ist, die vorzugsweise derart ausrichtbar ist, daß sich die von ihr ausgehenden therapeutischen Ultraschallwellen mit den von der Ultraschallquelle ausgehenden zumindest im Wirkbereich überlagern. Hierdurch laßt sich die Intensität der Ultraschallwellen im Wirkbereich im Interesse einer kurzen Behandlungsdauer erhöhen. Zumindest die von einer der Ultraschallquellen bzw. zumindest die von einem der therapeutischen Ultraschallwandler ausgehenden therapeutischen Ultraschallwellen sind auf eine Fokuszone fokussiert. Es ist dann möglich, die Verlagerung der Wirkzone in den jeweils zu behandelnden Bereich durch Verlagerung der Fokuszone innerhalb desjenigen Bereiches zu bewirken, indem sich die therapeutischen Ultraschallwellen überlagern. Besonders vorteilhaft ist es in diesem Zusammenhang, wenn eine größere und eine kleinere Fokuszone vorgesehen sind und die Verlagerung der Wirkzone durch Verlagerung der kleineren Fokuszone innerhalb der größeren erfolgt.

Gemäß einer Variante der Erfindung ist vorgesehen, daß zumindest eine der Ultraschallquellen bzw. zumindest einer der therapeutischen Ultraschallwandler die therapeutischen Ultraschallwellen pulsartig abstrahlt. Hierdurch wird erreicht, daß jeweils nur während der pulsartigen Abstrahlung die zur Erzielung eines therapeutischen Effektes erforderliche Intensität der therapeutischen Ultraschallwellen vorliegt.

Eine weitere Variante der Erfindung sieht vor, daß die Ultraschallwellen in der Wirkzone eine solche Energiedichte aufweisen und die Pulsdauer im Falle der pulsartigen Abstrahlung der therapeutischen Ultraschallwellen so gewählt ist, daß die während einer Herzaktion der Wirkzone zugeführte akustische Energie den gewünschten therapeutischen Effekt hervorruft.

Um eine Lokalisierung des jeweils zu behandelnden fokalen pathogenen Gebietes zu ermöglichen, ist es zweckmäßig, wenn Mittel zur Bestimmung der räumlichen Lage des zu behandelnden pathologischen Bereiches vorgesehen sind, die vorzugsweise eine Einrichtung zur Anfertigung von Magnetokardiogrammen, mittels derer Magnetfeldquellen räumlich lokalisierbar sind, oder eine Einrichtung für das EKG-Mapping enthalten. Es besteht dann die Möglichkeit, bei Auftreten der Symptome den Herd zu lokalisieren, von dem diese ausgehen, und den Herd anschließend zu behandeln.

Um eine effektive Kontrolle des Therapieprozesses zu ermöglichen, ist es zweckmäßig, wenn Mittel zur Bestimmung der Temperatur im Wirkbereich vorgesehen sind, die vorzugsweise eine Einrichtung zur zumindest qualitativen Bestimmung der Temperatur aus aufeinanderfolgenden Ultraschallbildern enthalten.

Ein Ausführungsbeispiel der Erfindung ist in den beigefügten Zeichnungen dargestellt. Es zeigen:
- Fig. 1: in teilweise blockschaltbildartiger, grob schematischer Darstellung ein erfindungsgemäßes Therapiegerät, das an einen in einem Sagittalschnitt dargestellten menschlichen Körper appliziert ist,
- Fig. 2: das an den in einem Transversalschnitt dargestellten Körper applizierte Gerät gemäß Fig. 1,
- Fig. 3: die ösophageal applizierbare Ultraschallquelle des Gerätes gemäß den Fig. 1 und 2 in einem Längsschnitt, und
- Fig. 4 bis 6: Schnitte gemäß den Linien IV bis VI in Fig. 3.

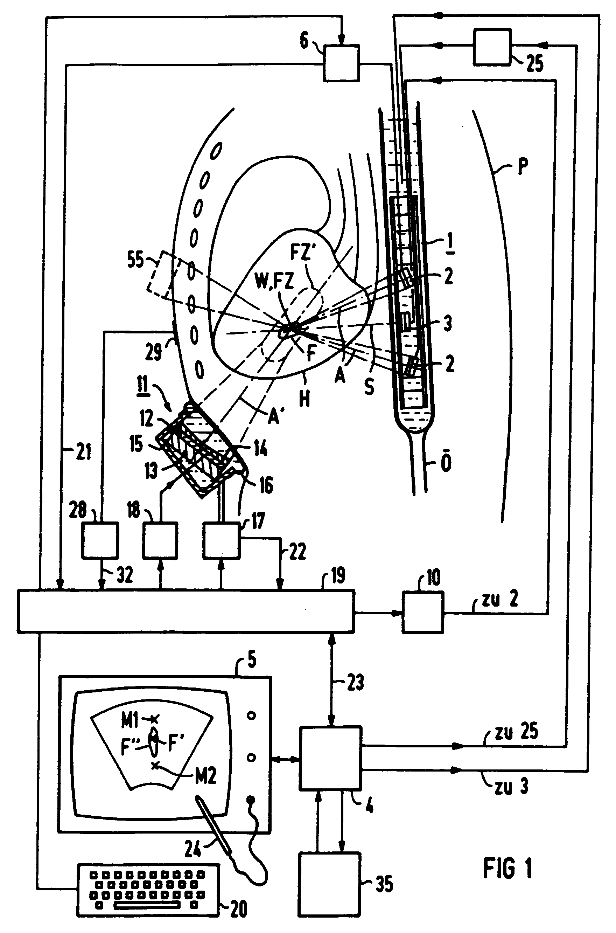

Die in Fig. 1 dargestellte Therapieeinrichtung weist eine durch den mit Ö bezeichneten Ösophagus (Speiseröhre) des Patienten P applizierbare Ultraschallquelle 1 auf, die mit einem flüssigen akustischen Ausbreitungsmedium, beispielsweise Wasser, gefüllt ist und die zwei therapeutische Ultraschallwandler 2 enthält. Die Ultraschallquelle 1 ist in dem Ösophagus so positioniert, daß sich die therapeutischen Ultraschallwandler 2 dicht bei dem zu behandelnden Herz H befinden.

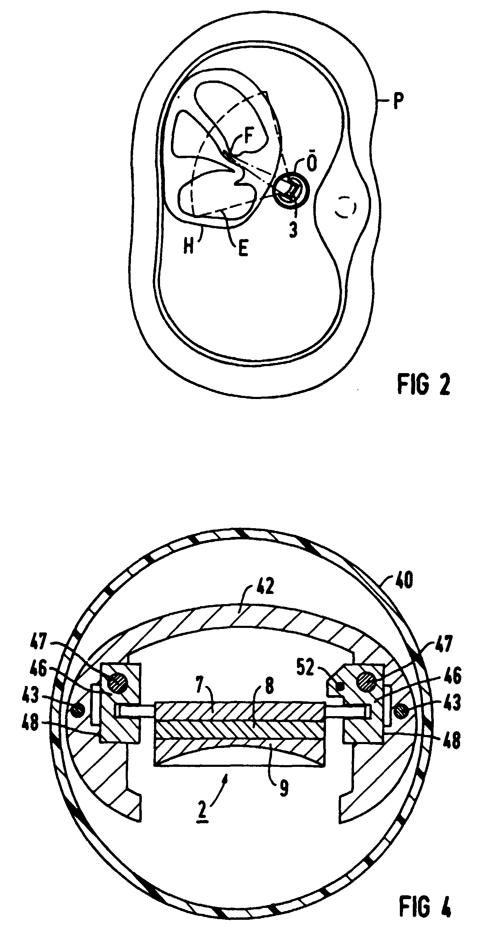

Die Ultraschallquelle 1 enthält außerdem einen diagnostischen Ultraschallwandler 3, der Bestandteil eines Ultraschall-Ortungssystems ist und dazu dient, den jeweils zu behandelnden Bereich, beispielsweise einen pathologischen Herd F, der Ausgangspunkt von Herzrythmusstörungen ist, zu lokalisieren. Der diagnostische Ultraschallwandler 3 ist mittels einer Antriebseinrichtung 25 um eine in der Zeichenebene liegend Achse derart schwenkbar, daß er im Zusammenwirken mit einer Steuerungs- und Bilderzeugungselektronik 4 in der Lage ist, Ultraschall-B-Bilder einer rechtwinklig zur Zeichenebene stehenden Ebene E (siehe Fig. 2) anzufertigen und auf einem Monitor 5 anzuzeigen. In die Fig. 1 ist die Schnittgerade S der abgetasteten Ebene mit der Zeichenebene eingetragen.

Bei der Durchführung einer Behandlung senden die therapeutischen Ultraschallwandler 2 therapeutische Ultraschallwellen aus. Die therapeutischen Ultraschallwandler 2 sind in der Ultraschallquelle 1 derart um rechtwinklig zur Zeichenebene stehende Achsen schwenkbar aufgenommen, daß sie mit Hilfe einer Verstelleinrichtung 6 unter Heranziehung des mittels des diagnostischen Ultraschallwandlers 3 und der Steuerungs- und Bilderzeugungseinheit 4 auf dem Monitor 5 dargestellten Ultraschallbildes zur Durchführung einer Behandlung derart ausrichtbar sind, daß sich die von den beiden therapeutischen Ultraschallwandlern 2 ausgehenden therapeutischen Ultraschallwellen, deren Randstrahlen strichliert angedeutet sind, in dem in der Ebene E plazierten zu behandelnden Herd F überlagern.

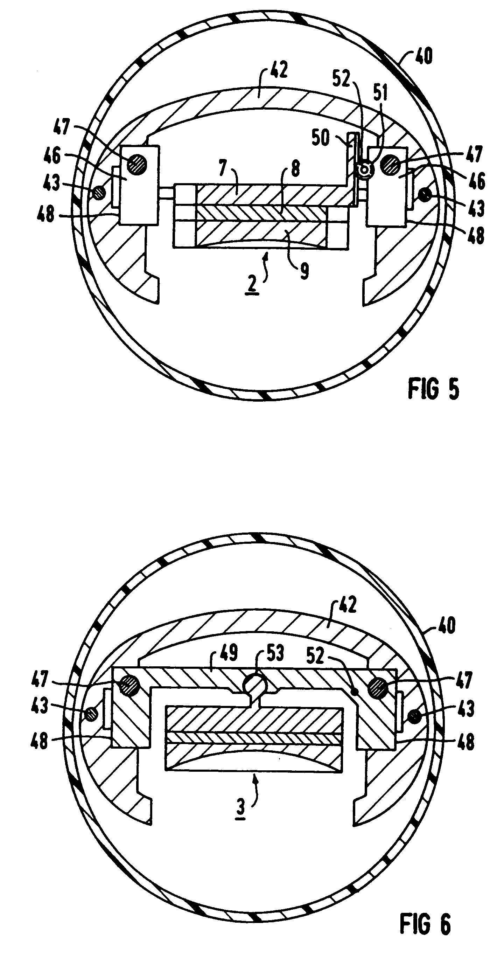

Bei den therapeutischen Ultraschallwandlern 2 handelt es sich um piezoelektrische Wandler, die gemäß Fig. 4 jeweils ein auf einem Backing (Tragkörper) 7 angebrachtes piezoelektrisches Wandlerelement 8 mit vorgesetzter akustischer Sammellinse 9 enthalten. Durch die akustischen Sammellinsen 9 werden die von den Wandlerelementen erzeugten akustischen Wellen auf eine Fokuszone FZ fokussiert.

Bei der Durchführung einer Behandlung sind die therapeutischen Ultraschallwandler 2 derart ausgerichtet, daß sich ihre Fokuszonen FZ in dem zu behandelnden Herd F überlappen. Die Wandlerelemente 8 können in an sich bekannten Weise in eine Vielzahl von ringförmigen Zonen unterteilt sein, die zur Abgabe von therapeutischen Ultraschallwellen mit zeitlicher Verzögerung ansteuerbar sind, so daß es möglich ist, die Lage der jeweiligen Fokuszone FZ auf elektronische Weise längs der jeweiligen akustischen Achse A zu verlagern. Die Sammellinsen 9 können dann unter Umständen entfallen.

Den therapeutischen Ultraschallwandlern 2 ist eine Ansteuereinheit 10 zugeordnet, die die zum Betrieb der therapeutischen Ultraschallwandler 2 erforderlichen Ströme zur Verfügung stellt. Sofern die therapeutischen Ultraschallwandler 2 eine elektronisch verlagerbare Fokuszone FZ aufweisen, sind die hierzu erforderlichen elektronischen Einrichtungen in die Ansteuereinheit 10 integriert.

Zusätzlich zu der Ultraschallquelle 1 ist ein therapeutischer Ultraschallapplikator 11 vorgesehen, der extrakorporal, und zwar subkostal, d.h. unterhalb des Rippenbogens, applizierbar ist. Auch der Ultraschallapplikator 11 enthält ein piezoelektrisches Wandlerelement 12, das auf einem Backing 13 angebracht und mit einer akustischen Sammellinse 14 versehen ist. Das Wandlerelement 12 ist in nicht näher dargestellter Weise mitsamt dem Backing 13 und der Sammellinse 14 kardanisch schwenkbar in einem Gehäuse 15 angeordnet. Dieses ist mit einem flüssigen akustischen Ausbreitungsmedium, beispielsweise Wasser, gefüllt und an seinem Applikationsende mit einer Koppelmembran 16 versehen, mittels derer der Ultraschallapplikator 11 zur akustischen Koppelung an die Körperoberfläche des Patienten P anpreßbar ist.

Dem Ultraschallapplikator 11 ist eine Verstelleinrichtung 17 zugeordnet, die dazu dient, das Wandlerelement 12 samt Backing 13 und Sammellinse 14 so auszurichten, daß der Herd F innerhalb der von dem Wandlerelement 12 ausgehenden therapeutischen Ultraschallwellen, insbesondere innerhalb deren Fokuszone FZ', liegt. Die Fokuszone FZ' ist von etwa rotationselliptischer Gestalt und strichliert angedeutet.

Dem Ultraschallapplikator 11 ist eine Ansteuereinrichtung 18 zugeordnet, die diesen mit dem zur Erzeugung von therapeutischen Ultraschallwellen erforderlichen Strom versorgt.

Die Verstelleinheit 6 und die Ansteuereinheit 10 sowie die Verstelleinrichtung 17 und die Ansteuereinrichtung 18 stehen mit einer Steuereinheit 19 in Verbindung, an die eine Tastatur 20 angeschlossen ist, die der Bedienung der Therapieeinrichtung dient. Die Verstelleinheit 6 und die Verstelleinrichtung 17 enthalten Positionssensoren, beispielsweise induktive Weggeber, die einerseits der Stellung der therapeutischen Ultraschallwandler 2 entsprechende Signale und andererseits der räumlichen Ausrichtung des Wandlerelementes 12 relativ zu der Ultraschallquelle 1 entsprechende Signale über Leitungen 21 und 22 an die Steuereinheit 19 liefern. Ausgehend von diesen Signalen errechnet die Steuereinheit 19 zum einen die Lage des Zentrums desjenigen Bereiches, in dem sich die Fokuszonen FZ der therapeutischen Ultraschallwandler 2 überlagern und zum anderen die Lage desjenigen Punktes, in dem die akustische Achse A' des Ultraschallapplikators 11 die mittels des diagnostischen Ultraschallwandlers 3 abtastbare Ebene E schneidet. Entsprechende Signale gibt die Steuereinheit 19 über eine Leitung 23 an die Steuer- und Bilderzeugungselektronik 4, die entsprechende Marken M1 (Überlagerung der Fokuszonen FZ) und M2 (Schnittpunkt A' mit E) in das Ultraschallbild einblendet, so wie dies in Fig. 2 angedeutet ist.

Zur Durchführung einer Behandlung werden über die Tastatur 20 die Verstelleinheit 6 und die Verstelleinrichtung 17 derart betätigt, daß sich die Marken M1 und M2 innerhalb des Abbildes F' des Herdes F in dem Ultraschallbild befinden. Ist dies der Fall, kann mit der Behandlung begonnen werden, wobei unterschiedliche, mittels der Tastatur 20 wahlbare Betriebsarten möglich sind.

Für die unterschiedlichen Betriebsarten ist wesentlich, daß die von dem Ultraschallapplikator 11 ausgehenden therapeutischen Ultraschallwellen relativ schwach fokussiert sind. Strahlt der Ultraschallapplikator 11 beispielsweise Ultraschall einer Frequenz von 875 kHz ab und weist einen Durchmesser von 3 cm auf, so ergibt sich ohne weitere Maßnahmen zur Fokussierung im Abstand von etwa 10 cm eine "natürliche" Fokuszone FZ' mit einem Durchmesser von etwa 10 mm. Die von den Ultraschallwandlern 2 der Ultraschallquelle 1 abgestrahlten therapeutischen Ultraschallwellen sind demgegenüber relativ stark fokussiert. So ergibt sich beispielsweise für einen Durchmesser der Ultraschallwandler 2 von 2 cm und eine Frequenz der von den Ultraschallwandlern 2 abgestrahlten Ultraschallwellen von 2 MHz jeweils eine Brennweite von 4 cm und ein Durchmesser der "natürlichen" Fokuszone FZ von 1,8 mm und eine Länge der Fokuszone von 20 mm. Damit kann das Zentrum desjenigen Bereiches, in dem sich die beiden Fokuszonen FZ überlagern, den Ultraschallwandlern 2 bis auf drei Zentimeter genähert bzw. von diesen bis auf fünf Zentimeter entfernt werden. Es versteht sich, daß sich durch die akustischen Sammellinsen 14 bzw. 9 jeweils eine schärfere Fokussierung ergibt bzw. daß bei Verwendung von Ultraschallwandlern 2 veränderlicher Brennweite andere Werte möglich sind. Wesentlich ist jedenfalls die unterschiedliche Größe der Fokuszonen FZ und FZ'. Die vorstehend genannten Abmessungen der Fokuszonen FZ und FZ' beziehen sich übrigens auf die sogenannte -6 dB-Isobare, d.h. als Fokuszone wird derjenige Bereich angesehen, in dem der Schalldruck der Ultraschallwellen wenigstens gleich der Hälfte des in der Fokuszone maximal auftretenden Schalldruckes ist.

In der ersten der genannten Betriebsarten stellt die Steuereinheit 19 durch entsprechende Einwirkung auf die Ansteuereinheit 10 die Intensität der von den Ultraschallwandlern 2 erzeugten Ultraschallwellen derart ein, daß die im Bereich der Überlagerung der Fokuszonen FZ resultierende Intensität noch nicht ausreicht, um den gewünschten therapeutischen Effekt, beispielsweise eine Nekrotisierung, zu bewirken. Die Intensität der von dem Ultraschallapplikator 11 ausgehenden Ultraschallwellen stellt die Steuereinheit 19 durch entsprechende Ansteuerung der Ansteuereinrichtung 18 derart ein, daß innerhalb der Fokuszone der von dem Ultraschallapplikator 11 ausgehenden therapeutischen Ultraschallwellen bei Überlagerung mit den von den Ultraschallwandlern 2 ausgehenden therapeutischen Ultraschallwellen eine zur Erzielung des gewünschten therapeutischen Effektes ausreichende Intensität erreicht wird. Dieser Bereich, bei dem es sich um den therapeutischen Wirkbereich W der Therapieeinrichtung handelt, kann dann innerhalb der Fokuszone FZ' des Ultraschallapplikators 11 dadurch verlagert werden, daß allmählich die Ausrichtung der Ultraschallwandler 2 relativ zueinander derart verändert wird, daß der Wirkbereich W in einer Abtastbewegung den gesamten innerhalb der Enden E liegenden Teil des Herdes F erfaßt. Dieser Vorgang kann anhand der in das Ultraschallbild eingeblendeten Marken M1 und M2 verfolgt werden. Die Abstrahlung der therapeutischen Ultraschallwellen erfolgt dabei zweckmäßigerweise nicht kontinuierlich, sondern wenigstens bezüglich des Ultraschallapplikators 11 oder eines der Ultraschallwandler 2 pulsartig, so daß nur dann, wenn zusätzlich zu einer eventuellen kontinuierlichen Abstrahlung von therapeutischen Ultraschallwellen auch die pulsartige Abstrahlung erfolgt, ein therapeutischer Effekt auftreten kann. Dabei erfolgt die Änderung der Ausrichtung der therapeutischen Ultraschallwandler 2 relativ zueinander manuell oder mittels der Steuereinheit 19 jeweils zwischen aufeinanderfolgenden pulsartigen Abstrahlungen.

Im Falle der Verlagerung des Wirkbereiches W mittels der Steuereinheit 19 kann vorgesehen sein, daß mittels eines Lichtgriffels 24 in dem Ultraschallbild ein zu behandelnder Bereich markiert wird, worauf die Steuereinheit 19 durch entsprechende Betätigung der Verstelleinrichtung 6 und eventuell der Verstelleinrichtung 17 den Wirkbereich W innerhalb der Markierung verlagert.

Eine weitere Betriebsart ist für Behandlungsfälle vorgesehen, in denen sich der Herd F dicht beim Ösophagus Ö befindet. In solchen Fallen reicht (unter Umständen) bereits die mittels der Ultraschallwandler 2 der Ultraschallquelle 1 realisierbare Ultraschallintensität zur Erzielung des gewünschten therapeutischen Effektes aus, so daß in dieser Betriebsart die Aktivierung des Ultraschallapplikators 11 unterbleibt.

In Fallen, in denen sich der zu behandelnde Bereich in einer größeren Entfernung vom Ösophagus Ö befindet, wird eine dritte Betriebsart gewählt, in der zusätzlich zu den Ultraschallwandlern 2 der Ultraschallquelle 1 und zu dem Ultraschallapplikator 11 auch der diagnostische Ultraschallwandler 3 zur Abstrahlung therapeutischer Ultraschallwellen genutzt wird, und zwar während der Intervalle, die zwischen der Erstellung der Ultraschallbilder liegen. Es läßt sich dann auch in relativ entfernt vom Ösophagus befindlichen Bereichen noch eine zur Erzielung eines therapeutischen Effektes ausreichende Ultraschallintensität realisieren. Die entsprechende mit der Steuereinheit 19 verbundene Ansteuereinheit für den diagnostischen Ultraschallwandler 3 ist in FIG 1 nicht dargestellt.

Die Behandlung des Herdes F mit therapeutischen Ultraschallwellen kann infolge der mechanischen Bewegung des Herzens H infolge der Herztätigkeit nicht zu beliebigen Zeitpunkten erfolgen. Vielmehr ist eine mit der Herztätigkeit synchronisierte Triggerung der therapeutischen Ultraschallwellen bzw. der pulsartigen Abstrahlung erforderlich. Zu diesem Zweck ist ein schematisch angedeutetes Kardiographiegerät 28 mit einer Elektrode 29 vorgesehen, das ein der Herztätigkeit entsprechendes elektrisches Signal an die Steuereinheit 19 gibt, die eine pulsartige Behandlung mit therapeutischem Ultraschall dann triggert, wenn sich das Herz in relativer mechanischer Ruhe befindet. Dies geschieht vorzugsweise in der Diastole, und zwar in dem Zeitraum zwischen dem Ende der T-Welle und dem Beginn des QRS-Komplexes. Bei einer Herzfrequenz von 75 Schlägen pro Minute dauert dieser Zeitraum etwa 0,5 sec.

Die Steuereinheit 19 stellt die Intensitäten der von den Ultraschallwandlern 2 und gegebenenfalls dem Ultraschallapplikator 11 so wie eventuell dem diagnostischen Ultraschallwandler 3 ausgehenden therapeutischen Ultraschallwellen derart ein, daß sich im Wirkbereich W eine Energiedichte der therapeutischen Ultraschallwellen ergibt, die ausreicht, um bereits während einer Herzaktion in dem jeweils im Wirkbereich W befindlichen Gewebe den gewünschten therapeutischen Effekt hervorzurufen.

Es besteht übrigens die Moglichkeit, bei Auftreten von pathologischen Erscheinungen mittels EKG-Mapping oder mit Hilfe eines Magnetokardiographiegerätes die räumliche Position des jeweiligen pathogenen Herdes F zu bestimmen. Entsprechende Daten können vor der Behandlung mittels der Tastatur 20 eingegeben werden. Die Steuereinheit 19 errechnet anhand dieser Daten diejenige Position der Ultraschallquelle 1, in der sich der Herd F innerhalb der mittels des diagnostischen Ultraschallwandlers 3 abtastbaren Ebene befindet und betätigt die Verstelleinheit 6 derart, daß diese die Ultraschallquelle 1 innerhalb des Ösophagus Ö in die entsprechende Position bringt und die therapeutischen Ultraschallwandler 2 entsprechend ausrichtet. Außerdem gibt die Steuereinheit 19 ein Signal an die Steuerungs- und Bilderzeugungselektronik 4, das dazu dient, in das Ultraschallbild eine Marke F'' einzublenden, die die eingegebene Position des Herdes F kennzeichnet. Dies ist insbesondere dann von Vorteil, wenn der Herd F in dem Ultraschallbild nicht ohne weiteres erkennbar ist.

Der Steuerungs- und Bilderzeugungselektronik 4 ist eine Auswerteelektronik 35 zugeordnet, die dazu dient, zu gleichen Zeitpunkten aufeinanderfolgender Herzaktionen erstellte Ultraschallbilder zu vergleichen, um in an sich bekannter Weise durch diesen Vergleich Temperaturerhöhungen zu detektieren. Durch Vergleich der ermittelten Temperaturerhöhung mit einem Schwellwert, der der für den gewünschten therapeutischen Effekt erforderlichen Temperatur entspricht, lassen sich diejenigen Bereiche detektieren, in denen zwischen zwei aufeinanderfolgenden Ultraschallbildern eine für den gewünschten therapeutischen Effekt ausreichende Temperaturerhöhung erreicht wurde. Die entsprechenden Daten gibt die Auswerteelektronik 35 an die Steuerungs- und Bilderzeugungselektronik 4, die die entsprechende Stelle im aktuellen Ultraschallbild markiert, beispielsweise durch eine abweichende Farbgebung. Auf diese Weise ist eine gute Überwachung des Therapievorganges möglich, wobei durch Beugungs- und Brechungserscheinungen in dem Gewebe des Patienten verursachte Mißweisungen der Ultraschall-Ortungseinrichtung und Abweichungen zwischen der tatsächlichen und der theoretischen Lage des Wirkbereiches der therapeutischen Ultraschallwellen ohne Einfluß sind.

Im folgenden wird der Aufbau der Ösophageal applizierbaren Ultraschallquelle 1 näher beschrieben. Gemäß den Fig. 1 bis 6 weist die Ultraschallquelle 1 eine flexible, schlauchartige Hülle 40 auf. Diese ist an ihrem distalen Ende verschlossen und enthält das akustische Ausbreitungsmedium für die von den therapeutischen Ultraschallwandlern 2 und dem diagnostischen Ultraschallwandler 3 ausgehenden Ultraschallwellen.

Innerhalb der Hülle 40 befindet sich eine insgesamt mit 41 bezeichnete Kettenstruktur 41, deren Glieder 42 wenigstens im Bereich der therapeutischen Ultraschallwandler 2 und des diagnostischen Ultraschallwandlers 3 einen C-förmigen Querschnitt aufweisen. In den übrigen Bereichen der Kettenstruktur können die Glieder 42 einen ovalen Querschnitt aufweisen. Die Glieder 42 sind mit parallel zu ihren Längsachsen verlaufenden, einander gegenüberliegenden Bohrungen versehen, die zur Aufnahme der Zugorgane 43 zweier Bowdenzüge dienen. Die Zugorgane 43 sind mit Klemmstücken 56 versehen, mit denen sie an der dem distalen Ende der Hülle 40 benachbarten Stirnfläche des entsprechenden Gliedes 42 anliegen. Die Umhüllungen 44 der Bowdenzüge stützen sich an der dem proximalen Ende der Hülle 40 zugewandten Stirnfläche des dem proximalen Ende der Hülle 40 am nächsten angeordneten Glied 42 ab.

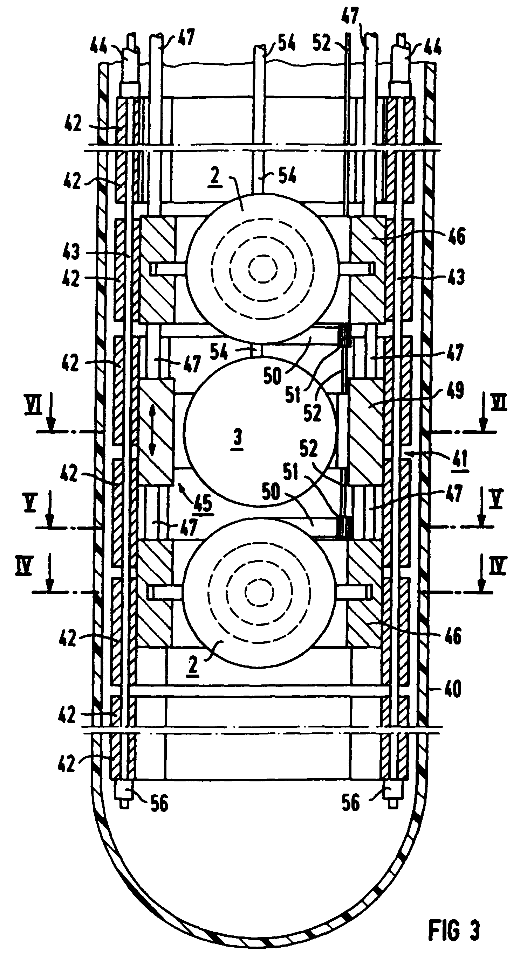

Den Bowdenzügen sind nicht dargestellte Spannmittel zugeordnet. Befinden sich diese im gelösten Zustand, kann zwischen einander benachbarten Gliedern 42 jeweils, so wie dies in Fig. 3 dargestellt ist, ein Abstand vorliegen, so daß die Kettenstruktur 41 flexibel ist. Die Ultraschallquelle 1 kann dann leicht in den Ösophagus des zu behandelnden Patienten P eingeführt werden. Werden die Spannmittel betätigt, liegen die Glieder 42 mit ihren planparallelen Stirnflächen aneinander an, so daß die Kettenstruktur 41 die in Fig. 1 angedeutete gradlinige Gestalt annimmt.

Innerhalb der Glieder 42 der Kettenstruktur 41 befindet sich eine Schlittenstruktur 45, die die therapeutischen Ultraschallwandler 2 und den diagnostischen Ultraschallwandler 3 tragt. Im einzelnen sind zwei Gleitschuhe 46 vorgesehen, die durch zwei flexible Drähte 47 miteinander verbunden sind. Mit jedem der Gleitschuhe 46 ist einer der therapeutischen Ultraschallwandler 2 in noch näher zu erläuternder Weise verbunden. Mittig zwischen den Gleitschuhen 46, die in der aus Fig. 4 ersichtlichen Weise in Gleitbahnen 48 der Glieder 42 geführt sind, ist ein Tragteil 49 angeordnet, an dem der diagnostische Ultraschallwandler 3 befestigt ist. Mittels der Drähte, auf die die Verstelleinheit 6 einwirkt, ist die Schlittenstruktur 45 innerhalb der Kettenstruktur 41 längsverschieblich, was durch einen entsprechenden Doppelpfeil angedeutet ist.

Die Backings 7 der therapeutischen Ultraschallwandler 2 weisen in entsprechenden Bohrungen der Gleitschuhe 46 aufgenommene Zapfen auf, so daß die therapeutischen Ultraschallwandler 2 in der bereits zuvor beschriebenen Weise schwenkbar sind. An jedem der therapeutischen Ultraschallwandler ist ein Zahnradsegment 50 angebracht, das jeweils mit einem entsprechenden Ritzel 51 zusammenwirkt. Die Ritzel 51 sind auf einem in entsprechenden Bohrungen der Gleitschuhe 46 drehbar gelagerten flexiblen Draht 52 drehfest angebracht.

Die Zahnradsegmente 50 und die Ritzel 51 sind derart dimensioniert, und die Zahnradsegmente 50 derart an den therapeutischen Ultraschallwandlern 2 angebracht, daß dann, wenn der Draht 52 mittels der Verstelleinheit 6 verdreht wird, eine synchrone Schwenkbewegung der therapeutischen Ultraschallwandler 2 aufeinander zu bzw. voneinander weg erfolgt.

Der diagnostische Ultraschallwandler 3 ist an dem Tragteil 49 mittels eines Schwenklagers 53 derart schwenkbar gelagert, daß die zuvor angegebene Körperschicht des Patienten P abtastbar ist. Die Schwenkbewegung wird mittels eines flexiblen Drahtes 54 von der Antriebseinrichtung 25 auf den diagnostischen Ultraschallwander 3 übertragen.

Die zu den Ultraschallwandlern 2 und 3 führenden elektrischen Leitungen sind in den Fig. 4 bis 6 der Einfachheit halber nicht dargestellt. In Fig. 4 ist übrigens durch strichlierte Kreise angedeutet, daß es sich bei den therapeutischen Ultraschallwandlern 2 um elektronisch fokussierbare Ringstrukturen handeln kann.

In manchen Behandlungsfällen kann es zweckmäßig sein, zusätzlich zu der Ultraschallquelle 1 und dem Ultraschallapplikator 11 noch einen weiteren extrakorporal applizierbaren Ultraschallapplikator 55 vorzusehen. Dieser ist in der Fig. 1 strichliert angedeutet. Er wird interkostal appliziert, d.h., er strahlt durch zwei einander benachbarte Rippen des Patienten P hindurch. Die von dem Ultraschallapplikator 55 abgestrahlten therapeutischen Ultraschallwellen sind vorzugsweise schwach fokussiert. Die Ansteuerung und die Ausrichtung des Ultraschallapplikators 55 erfolgen in nicht dargestellter Weise analog zu den im Falle des Ultraschallapplikators 11 vorliegenden Verhältnissen.

Im Falle des beschriebenen Ausführungsbeispieles sind sowohl eine transösophageal applizierbare Ultraschallquelle als auch wenigstens ein extrakorporal applizierbarer Ultraschallapplikator vorhanden. Im Rahmen der Erfindung kann jedoch vorgesehen sein, daß die Therapieeinrichtung entweder nur eine transösophageal applizierbare Ultraschallquelle oder alternativ nur extrakorporal applizierbare Ultraschallapplikatoren bzw. einen einzigen solchen Ultraschallapplikator aufweist.

Im Falle des vorstehend beschriebenen Ausführungsbeispieles wird ein pathogener Herd F im Bereich des Herzens H selbst behandelt. Es versteht sich, daß mittels erfindungsgemäßer Therapieeinrichtungen auch (Blut-)Gefäße, insbesondere herznahe Gefäße, behandelbar sind.

## Patentansprüche

1. Therapieeinrichtung zur Behandlung von Leiden des Herzens (H) und Gefäßen, einschließlich herznaher Gefäße, mit einen Wirkbereich (W) aufweisenden therapeutischen Ultraschallwellen, welche Therapieeinrichtung eine therapeutische Ultraschallquelle (1, 11, 55) aufweist, die therapeutische Ultraschallwellen einer solchen Intensität erzeugt, daß in dem im Wirkbereich (W) befindlichen Gewebe Gewebeveränderungen durch thermische Wirkung hervorgerufen werden, und die mehrere therapeutische Ultraschallwandler (2) enthält, welche derart ausrichtbar sind, daß sich die von ihnen ausgehenden Ultraschallwellen in dem jeweils zu behandelnden Bereich überlagern, **dadurch gekennzeichnet,** daß die Ultraschallquelle (1, 11, 55) derart dimensioniert ist, daß sie transösophageal applizierbar ist.

2. Therapieeinrichtung nach Anspruch 1, **dadurch gekennzeichnet,** daß die Intensität der therapeutischen Ultraschallwellen ausreicht, um Nekrotisierungen des im Wirkbereich (W) befindlichen Gewebes hervorzurufen.

3. Therapieeinrichtung nach Anspruch 1, **dadurch gekennzeichnet,** daß die Ultraschallquelle (1, 11, 55) therapeutische Ultraschallwellen einer solchen Intensität erzeugt, daß in dem im Wirkbereich (W) befindlichen Gewebe bleibende Gewebeveränderungen durch thermische Wirkung hervorgerufen werden.

4. Therapieeinrichtung nach einem der Ansprüche 1 bis 3, **dadurch gekennzeichnet,** daß die Ultraschallquelle (1) einen diagnostischen Ultraschallwandler (3) enthält, der Bestandteil einer Ultraschall-Ortungseinrichtung zur Ortung des jeweils zu behandelnden Bereiches ist.

5. Therapieeinrichtung nach Anspruch 3 oder 4, **da durch gekennzeichnet,** daß die therapeutischen Ultraschallwandler (2) und gegebenenfalls der diagnostische Ultraschallwandler (3) Bestandteile einer kettenartigen Struktur (41) sind, wobei die Struktur (41) in nicht arretiertem Zustand flexibel und in arretiertem Zustand wenigstens im wesentlichen starr ist.

6. Therapieeinrichtung nach Anspruch 3 oder 4, **da durch gekennzeichnet,** daß die therapeutischen Ultraschallwandler (2) und gegebenenfalls der diagnostische Ultraschallwandler (3) an Gliedern einer eine Anzahl aufeinanderfolgender Glieder aufweisenden Struktur (41) sind, wobei die Struktur (41) in nicht arretiertem Zustand flexibel und in arretiertem Zustand wenigstens im wesentlichen starr ist.

7. Therapieeinrichtung nach einem der Ansprüche 1 bis 6, **dadurch gekennzeichnet,** daß Mittel (28, 29) vorgesehen sind, die eine Abgabe therapeutischer Ultraschallwellen nur wahrend solcher Phasen der Herztätigkeit freigeben, in denen sich das Herz (H) in relativer mechanischer Ruhe befindet.

8. Therapieeinrichtung nach einem der Ansprüche 1 bis 7, **dadurch gekennzeichnet,** daß wenigstens eine extrakorporal, insbesondere inter- oder subkostal, applizierbare therapeutische Ultraschallquelle (11, 55) vorgesehen ist.

9. Therapieeinrichtung nach Anspruch 8, **dadurch gekennzeichnet,** daß die extrakorporal applizierbare Ultraschallquelle (11, 55) derart ausrichtbar ist, daß sich die von ihr ausgehenden therapeutischen Ultraschallwellen mit den von einer anderen Ultraschallquelle (1, 55 bzw. 1, 11) ausgehenden, zumindest im Wirkbereich (W) überlagern.

10. Therapieeinrichtung nach einem der Ansprüche 1 bis 9, **dadurch gekennzeichnet,** daß zumindest die von einer Ultraschallquelle (1, 11, 55) bzw. zumindest die von wenigstens einem therapeutischen Ultraschallwandler (2) ausgehenden Ultraschallwellen auf eine Fokuszone (FZ, FZ') fokussiert sind.

11. Therapieeinrichtung nach Anspruch 10, **dadurch gekennzeichnet,** daß eine größere und eine kleinere Fokuszone (FZ', FZ) vorgesehen sind, und daß die Verlagerung der Wirkzone (W) in dem jeweils zu behandelnden Bereich durch Verlagerung der kleineren Fokuszone (FZ) innerhalb der größeren (FZ') erfolgt.

12. Therapieeinrichtung nach einem der Ansprüche 1 bis 11, **dadurch gekennzeichnet,** daß zumindestens eine der therapeutischen Ultraschallquellen (1, 11, 55) bzw. zumindestens wenigstens einer der therapeutischen Ultraschallwandler (2) die therapeutischen Ultraschallwellen pulsartig abstrahlt.

13. Therapieeinrichtung nach Anspruch 12, **dadurch gekennzeichnet,** daß die Ultraschallwellen in der Wirkzone (W) eine solche Energiedichte aufweisen und die Pulsdauer bei der pulsartigen Abstrahlung der therapeutischen Ultraschallwellen so gewählt ist, daß die wahrend einer Herzaktion zugeführte akustische Energie den therapeutischen Effekt hervorruft.

14. Therapieeinrichtung nach einem der Ansprüche 1 bis 13, **dadurch gekennzeichnet,** daß Mittel zur Bestimmung der Temperatur im Wirkbereich vorgesehen sind, die vorzugsweise eine Einrichtung (35) zur zumindestens qualitativen Bestimmung der Temperatur aus aufeinanderfolgenden Ultraschallbildern enthalten.

## Claims

1. Therapy apparatus for treating conditions of the heart (H) and vessels, including vessels near the heart, with therapeutic ultrasound waves having an effective region (W), which therapy apparatus has a therapeutic ultrasound source (1, 11, 55) which generates therapeutic ultrasound waves of such an intensity that tissue changes are brought about in the tissue located in the effective region (W) by means of a thermal effect and which contains a plurality of therapeutic ultrasonic transducers (2) which can be aligned in such a way that the ultrasound waves issuing from them are superimposed in the respective region to be treated, characterised in that the ultrasound source (1, 11, 55) is dimensioned in such a way that it can be applied through the oesophagus.

2. Therapy apparatus according to claim 1, characterised in that the intensity of the therapeutic ultrasound waves suffices to bring about instances of necrotization of the tissue located in the effective region (W).

3. Therapy apparatus according to claim 1, characterised in that the ultrasound source (1, 11, 55) generates therapeutic ultrasound waves of such an intensity that permanent tissue changes in the tissue located in the effective region (W) are brought about by means of a thermal effect.

4. Therapy apparatus according to one of claims 1 to 3, characterised in that the ultrasound source (1) contains a diagnostic ultrasonic transducer (3) which is part of an ultrasonic locating device for the purpose of locating the respective region to be treated.

5. Therapy apparatus according to claim 3 or 4, characterized in that the therapeutic ultrasonic transducers (2) and, if applicable, the diagnostic ultrasonic transducer (3) are parts of a chain-like structure (41), with the structure (41) being flexible in a non-arrested state and being at least substantially rigid in an arrested state.

6. Therapy apparatus according to claim 3 or 4, characterised in that the therapeutic ultrasonic transducers (2) and, if applicable, the diagnostic ultrasonic transducer (3) are on elements of a structure (41) which has a number of successive elements, with the structure (41) being flexible in a non-arrested state and being at least substantially rigid in an arrested state.

7. Therapy apparatus according to one of claims 1 to 6, characterized in that means (28, 29) are provided that enable therapeutic ultrasound waves to be emitted only during such phases of heart activity during which the heart (H) is in a state of relative mechanical rest.

8. Therapy apparatus according to one of claims 1 to 7, characterized in that at least one therapeutic ultrasound source (11, 55) that can be applied extracorporeally, in particular intercostally or subcostally, is provided.

9. Therapy apparatus according to claim 8, characterised in that the ultrasound source (11, 55) that can be applied extracorporeally can be aligned in such a way that the therapeutic ultrasound waves issuing from it are superimposed with those issuing from another ultrasound source (1, 55 or 1, 11 respectively), at least in the effective region (W).

10. Therapy apparatus according to one of claims 1 to 9, characterised in that at least the ultrasound waves issuing from an ultrasound source (1, 11, 55) or at least the ultrasound waves issuing from at least one therapeutic ultrasonic transducer (2) are focussed on a focus zone (FZ, FZ').

11. Therapy apparatus according to claim 10, characterised in that a comparatively large and a comparatively small focus zone (FZ', FZ) are provided, and in that the displacement of the effective zone (W) in the respective region to be treated is effected by displacing the smaller focus zone (FZ) within the larger focus zone (FZ').

12. Therapy apparatus according to one of claims 1 to 11, characterised in that at least one of the therapeutic ultrasonic sources (1, 11, 55) or at least one of the therapeutic ultrasonic transducers (2) emits the therapeutic ultrasound waves in pulses.

13. Therapy apparatus according to claim 12, characterised in that the ultrasound waves in the effective zone (W) have such an energy density and the selected pulse duration in the case of the pulsed emission of the therapeutic ultrasound waves is such that the acoustic energy supplied during a heart action brings about the therapeutic effect.

14. Therapy apparatus according to one of claims 1 to 13, characterised in that means are provided in order to determine the temperature in the effective region, which means preferably contain a device (35) for at least qualitatively determining the temperature from successive ultrasonic images.

## Revendications

1. Dispositif pour le traitement de maladies du coeur (H) et de vaisseaux, y compris de vaisseaux proches du coeur, à l'aide d'ondes ultrasonores thérapeutiques ayant une zone (W) d'action, ce dispositif de traitement comportant une source (1, 11, 55) ultrasonore thérapeutique, qui produit des ondes ultrasonores thérapeutiques d'une intensité telle que des modifications sont produites par effet thermique dans le tissu se trouvant dans la zone (W) d'action, et qui comprend plusieurs transducteurs (2) ultrasonores thérapeutiques qui peuvent être orientés de telle sorte que les ondes ultrasonores qui en proviennent se superposent dans la zone respective à traiter, **caractérisé** en ce que la source (1, 11, 55) ultrasonore est dimensionnée de telle sorte qu'elle peut être mise en place en passant par l'oesophage.

2. Dispositif de traitement suivant la revendication 1, **caractérisé** en ce que l'intensité des ondes ultrasonores thérapeutiques est suffisante pour produire des nécroses du tissu se trouvant dans la zone (W) d'action.

3. Dispositif de traitement suivant la revendication 1, **caractérisé** en ce que la source (1, 11, 55) ultrasonore produit des ondes ultrasonores thérapeutiques d'une intensité telle que des modifications permanentes sont produites par effet thermique dans le tissu se trouvant dans la zone (W) d'action.

4. Dispositif de traitement suivant l'une des revendications 1 à 3, **caractérisé** en ce que la source (1) ultrasonore comprend un transducteur (3) ultrasonore de diagnostic, qui fait partie d'un dispositif de localisation ultrasonore pour localiser la zone respective à traiter.

5. Dispositif de traitement suivant la revendication 3 ou 4, **caractérisé** en ce que le transducteur (2) ultrasonore thérapeutique, et le cas échéant le transducteur (3) ultrasonore de diagnostic, font partie d'une structure (41) du genre chaîne, la structure (41) étant flexible lorsqu'elle n'est pas bloquée, et au moins sensiblement rigide lorsqu'elle est bloquée.

6. Dispositif de traitement suivant la revendication 3 ou 4, **caractérisé** en ce que le transducteur (2) ultrasonore thérapeutique, et le cas échéant le transducteur (3) ultrasonore de diagnostic, se trouvent sur des maillons d'une structure (41) comportant un certain nombre de maillons successifs, la structure (41) étant souple lorsqu'elle n'est pas bloquée, et au moins sensiblement rigide lorsqu'elle est bloquée.

7. Dispositif de traitement suivant l'une des revendications 1 à 6, **caractérisé** en ce qu'il est prévu des moyens (28, 29) qui n'autorisent une émission d'ondes ultrasonores thérapeutiques que pendant les phases de l'activité cardiaque au cours desquelles le coeur (H) se trouve en repos mécanique relatif.

8. Dispositif de traitement suivant l'une des revendications 1 à 7, **caractérisé** en ce qu'il est prévu au moins une source (11, 55) ultrasonore thérapeutique qui peut être mise en place de façon extracorporelle, notamment de façon intercostale ou subcostale.

9. Dispositif de traitement suivant la revendication 8, **caractérisé** en ce que la source (11, 55) ultrasonore pouvant être mise en place de façon extracorporelle peut être orientée de telle sorte que les ondes ultrasonores thérapeutiques qui en proviennent se superposent, au moins dans la zone (W) d'action, à celles provenant d'une autre source (1, 55 ou 1, 11) ultrasonore.

10. Dispositif de traitement suivant l'une des revendications 1 à 9, **caractérisé** en ce qu'au moins les ondes ultrasonores qui proviennent d'une source (1, 11, 55) ultrasonore, ou encore au moins les ondes ultrasonores qui proviennent d'au moins un transducteur (2) ultrasonore thérapeutique, sont focalisées sur une zone (FZ, FZ') de focalisation.

11. Dispositif de traitement suivant la revendication 10, **caractérisé** en ce qu'il est prévu une zone (FZ') de focalisation plus grande et une zone (FZ) de focalisation plus petite, et en ce que le déplacement de la zone (W) d'action dans la zone respective à traiter s'effectue par déplacement de la plus petite zone (FZ) de focalisation à l'intérieur de la plus grande zone (FZ') de focalisation plus petite

12. Dispositif de traitement suivant l'une des revendications 1 à 11, **caractérisé** en ce qu'au moins une des sources (1, 11, 55) ultrasonores thérapeutiques, ou encore au moins un des transducteurs (2) ultrasonores thérapeutiques, émet les ondes ultrasonores thérapeutiques de façon pulsatoire.

13. Dispositif de traitement suivant la revendication 12, **caractérisé** en ce que les ondes ultrasonores possèdent dans la zone (W) d'action une densité d'énergie telle, et la durée d'impulsion lors de l'émission pulsatoire des ondes ultrasonores thérapeutiques est choisie telle, que l'énergie acoustique apportée pendant une activité cardiaque produit l'effet thérapeutique.

14. Dispositif de traitement suivant l'une des revendications 1 à 13, **caractérisé** en ce qu'il est prévu des moyens pour déterminer la température dans la zone d'action, moyens qui comprennent de préférence un dispositif (35) pour la détermination au moins qualitative de la température à partir d'images ultrasonores successives.
